# FASCICULE DE BREVET EUROPEEN

(11) **EP 2 922 498 B1**
(45) Date de publication et mention de la délivrance du brevet: **01.02.2017**
(21) Numéro de dépôt: 13792405.6
(22) Date de dépôt: 20.11.2013
(51) Int. Cl.: A61F 2/20, A61F 2/95

(54) **DISPOSITIF DE POSE ET DE DÉPOSE D'UNE PROTHÈSE**
VORRICHTUNG ZUR PASSUNG UND ENTFERNUNG EINER PROTHESE
DEVICE FOR FITTING AND REMOVING A PROSTHESIS

(30) Priorité: 20.11.2012 FR 1261029
(43) Date de publication de la demande: 30.09.2015
(73) Titulaire: Protip Medical, 67000 Strasbourg (FR)
(72) Inventeur: PERRIN, Nicolas, F-67300 Schiltigheim (FR)
(74) Mandataire: Flesselles, Bruno F.G.
(86) Numéro de dépôt international: PCT/EP2013/074322
(87) Numéro de publication internationale: WO 2014/079904

(56) Documents cités:
- WO-A1-96/35399
- WO-A1-2005/097001
- WO-A1-2011/051177
- DE-U1-202007 000 655
- FR-A1- 2 902 641
- US-B1- 6 565 581
- US-B1- 6 666 208

## Description

### DOMAINE TECHNIQUE

La présente invention concerne un dispositif de pose et de dépose d'une prothèse, qui est de préférence une prothèse intra laryngée, ainsi qu'un kit de pose et de dépose d'une prothèse, qui est également de préférence une prothèse intra laryngée.

### ETAT DE LA TECHNIQUE ANTERIEUR

La demande de brevet FR n°11 02694 décrit une prothèse intra laryngée qui présente une excellente résistance à l'usure et à la déformation en conditions physiologiques, ainsi qu'une durée de vie importante. Cette prothèse intra laryngée est réalisée en métal massif biocompatible et est destinée à être implantée au sein d'un larynx dysfonctionnel, ledit dispositif comportant une portion distale formant armature porteuse annulaire et une portion centrale formant obturateur destinée à permettre le passage de l'air et à empêcher de manière hermétique le passage de tout autre élément, l'obturateur comprenant i) une partie périphérique formant un premier clapet solidaire de l'armature porteuse annulaire au niveau d'une première région charnière, et ii) une partie centrale formant un second clapet solidaire du premier clapet au niveau d'une seconde région charnière, lesdits premier et second clapets coopérant entre eux de manière totalement hermétique.

Jusqu'à présent, cette prothèse intra laryngée était mise en place dans le larynx du patient manuellement par le chirurgien. Toutefois, cette pose manuelle était compliquée, longue et peu précise. Par ailleurs, il était compliqué, voire impossible de poser cette prothèse chez des patients n'ayant pas subi de trachéotomie. En effet, le mode opératoire pour la pose de la prothèse intralaryngée demandait que le chirurgien positionne correctement la prothèse en la « tirant *» via* le trou de trachéotomie. La mise en place de la prothèse chez des patients ayant un larynx partiellement dysfonctionnel (tel que des patients présentant des troubles de déglutition) demandait ainsi d'effectuer une trachéotomie avant correction du trouble, ce qui menait à un autre effet secondaire handicapant pour le patient.

On recherche donc à prévenir et traiter les troubles de dysfonctionnement du larynx en permettant le positionnement d'une prothèse permettant de rétablir un fonctionnement normal de la fonction laryngée, sans effectuer de trachéotomie chez le patient. La Demanderesse a donc développé un ancillaire de pose et dépose de telles prothèses, qui puisse être opéré directement et uniquement par voie endobuccale.

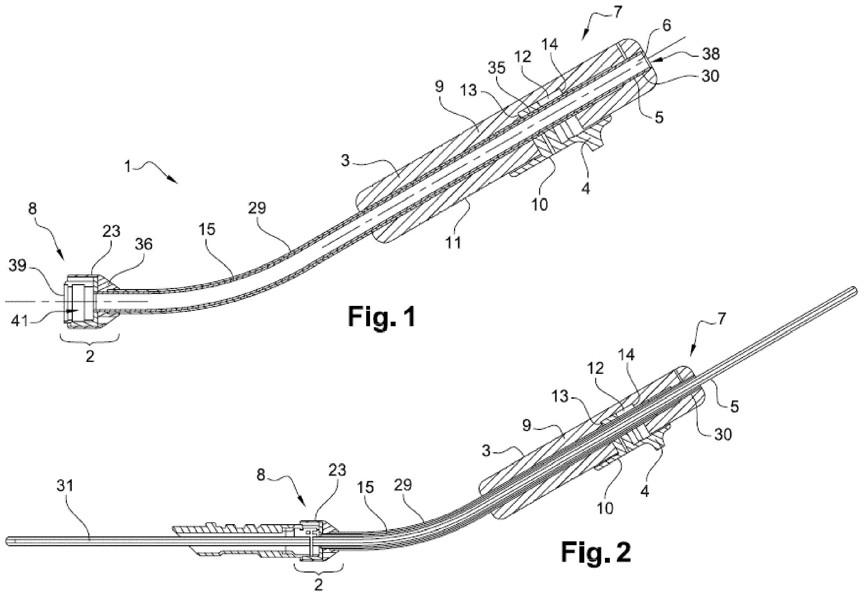

WO 96/35399 décrit une prothèse phonatoire, ainsi q'un ancillaire pour son implantation. La voie d'accès se fait par le trachéostome (figure 1). Ce dispositif utilise la rotation des éléments les uns par rapport aux autres et possède un axe de révolution. Les moyens de liaison ne sont pas flexibles, car devant permettre la déformation d'une partie de la prothèse pour implantation.

WO 2011/051177 décrit également un ancillaire pour l'implantation d'une prothèse phonatoire. La voie d'accès se fait par le trachéostome (figure 19). Ce dispositif utilise la rotation des éléments les uns par rapport aux autres et possède un axe de révolution. Les moyens de liaison n'apparaissent pas comme étant flexibles

WO 2005/097001 décrit un ancillaire décrit est pour l'implantation d'une prothèse à travers une paroi flexible qui n'est accessible que d'un seul côté. La voie d'accès se fait par le trou de trachéotomie présent après laryngectomie totale (page 3 lignes 15-19). Du fait du mode opératoire (il convient de pousser sur l'ancillaire (voir figure 14 à 16), le dispositif ne présente pas de partie flexible.

Ces trois dispositifs nécessite également l'utilisation des deux mains de l'opérateur. US 6666208 décrit un ancillaire pour l'implantation d'une prothèse phonatoire. La voie d'accès se fait par le trachéostome.(col.3, lines 1-2). Ce dispositif ne met pas en oeuvre de moyens de liaison au moins en partie flexibles.

US 6565581 décrit un dispositif pour réaliser une anastomose. Le dispositif de pose de stents ne présente pas de moyens de liaison au moins en partie flexibles.

### EXPOSE DE L'INVENTION

L'invention vise à remédier aux inconvénients de l'état de la technique en proposant un dispositif permettant de mettre en place facilement une prothèse, et notamment une prothèse intra laryngée, dans le corps d'un patient. Ce dispositif permet de travailler par voie endobuccale, et ne nécessite que l'utilisation d'une seule main de l'opérateur.

Pour ce faire, est proposé selon un premier aspect de l'invention, un dispositif de pose et de dépose d'une prothèse selon les revendications 1 à 9 comportant :
- Des moyens de fixation aptes à se fixer sur la prothèse, les moyens de fixation pouvant être placés dans deux positions :
   ∘ Une position fermée dans laquelle ils sont solidaires de la prothèse;
   ∘ Une position ouverte dans laquelle ils ne sont pas solidaires de la prothèse;
- Des moyens de préhension permettant de manipuler le dispositif de pose et de dépose;
- Des moyens de contrôle, les moyens de contrôle étant reliés aux moyens de fixation, les moyens de contrôle étant aptes à commander les moyens de fixation de façon à les placer dans la position fermée ou dans la position ouverte ;
- Des moyens de liaison, au moins en partie flexibles, qui relient les moyens de contrôle aux moyens de fixation de façon à transmettre la commande des moyens de contrôle aux moyens de fixation.

Les moyens de préhension sont destinés à rester à l'extérieur du patient et à permettre au chirurgien de manipuler la prothèse qui sera insérée dans le corps du patient depuis l'extérieur du corps du patient. Les moyens de liaison auront donc une longueur permettant aux moyens de préhension de rester à l'extérieur du corps du patient tandis que les moyens de fixation se trouvent dans le corps du patient, à l'endroit où la prothèse doit être posée. Les moyens de liaison seront donc destinés à avoir une extrémité à l'extérieur du patient, tandis que l'autre extrémité sera dans le corps du patient.

Les moyens de fixation ont une géométrie et des dimensions adaptées à la prothèse sur laquelle ils sont destinés à se fixer.

Le dispositif de pose et de dépose est destiné à la pose et la dépose d'une prothèse intra laryngée, les moyens de fixation sont aptes à être insérés dans un larynx par voie endobuccale. Pour cela, leurs dimensions extérieures sont de préférence inférieures à celles des conduits dans lesquels ils sont destinés à être insérés.

Ces moyens de fixation doivent essentiellement être rigides pour assurer leur fonction principale de bon positionnement et fixation de la prothèse.

On utilise ainsi notamment un matériau présentant d'excellentes propriétés mécaniques, électriques et à l'usure, ainsi qu'une bonne stabilité. Ce matériau a également, préférentiellement, un faible coefficient de frottement pour des utilisations dans des environnements humides et secs. On utilise notamment le POMALUX® (copolymère acétal, Westlake Plastics, Lenni, PA, États-Unis)

Les moyens de fixation doivent présenter un encombrement minimal notamment pour passer entre les dents du patient : le diamètre extérieur du composant le plus encombrant est donc de préférence inférieur à 25mm, de l'ordre de 24,30 mm +/- 0,1.

Lorsque les moyens de fixation sont dans la position fermée, ils emprisonnent la prothèse, ce qui permet de la déplacer jusqu'à l'endroit voulu dans le corps du patient. Une fois que la prothèse est dans la position voulue, les moyens de fixation peuvent être placés dans la position ouverte, dans laquelle ils libèrent la prothèse. Le dispositif de pose et de dépose peut alors être retiré du corps du patient, tandis que la prothèse reste en place. Le passage des moyens de fixation de la position fermée à la position ouverte, et inversement, est contrôlé depuis l'extérieur du corps du patient par les moyens de contrôle.

Les moyens de fixation permettent de sécuriser la pose de la prothèse puisque les moyens de fixation sont agencés de façon à ce que la prothèse ne puisse pas se détacher du dispositif de pose et de dépose tant que les moyens de fixation sont en position fermée.

Par ailleurs, les moyens de fixation sont de préférence conformés de façon à ce que la prothèse puisse être insérée dans les moyens de fixation lorsque, et seulement lorsque, les moyens de fixation sont en position ouverte.

Le dispositif selon l'invention permet donc de poser facilement et de manière sécurisée une prothèse.

En outre, il peut également être utilisé pour la dépose d'une prothèse. Pour ce faire, le dispositif de pose et de dépose est introduit dans le corps du patient jusqu'à ce qu'il atteigne la prothèse. Le moyens de fixation sont placés en position ouverte. Puis, lorsque les moyens de fixation sont dans la position voulue par rapport à la prothèse, ils sont placés en position fermée de façon à ce qu'ils se solidarisent avec la prothèse. Les moyens de fixation sont alors retirés du corps du patient, et ils entraînent avec eux la prothèse. Le dispositif de pose et de dépose selon l'invention permet donc de retirer facilement une prothèse du corps d'un patient.

Les moyens de préhension comportent de préférence un manche.

Les moyens de contrôle sont de préférence disposés sur les moyens de préhension de façon à faciliter l'utilisation du dispositif de pose et de dépose par un chirurgien. En effet, le chirurgien peut alors tenir les moyens de préhension d'une main, et contrôler la position des moyens de fixation via les moyens de contrôle avec la même main, par exemple avec le pouce de la même main.

Selon un mode de réalisation préférentiel, les moyens de fixation comportent :
- une zone d'assemblage apte à être assemblée avec une partie de la prothèse ;
- Une dent déformable élastiquement pourvue de moyens de retenue aptes à être retenus par une partie complémentaire de la prothèse, la dent devant se déformer élastiquement dans au moins une direction dite « direction de libération » pour permettre aux moyens de retenue de se libérer de la partie complémentaire de la prothèse ;
- Des moyens de blocage pouvant être placés dans deux positions :
   ∘ Une position fermée dans laquelle ils empêchent la déformation de la dent dans la direction de libération ;
   ∘ Une position ouverte dans laquelle ils n'empêchent pas la déformation de la dent dans la direction de libération.

La zone d'assemblage permet d'assembler la prothèse et le dispositif de pose et de dépose. Toutefois, en l'absence de la dent et des moyens de retenue, la prothèse peut se désolidariser facilement du dispositif de pose et de dépose. La dent pourvue des moyens de retenue permet de lier la prothèse au dispositif de pose et de dépose. Cette liaison peut être défaite lorsque les moyens de blocage sont dans la position ouverte. Par contre, tant que les moyens de blocage sont dans la position fermée, la prothèse ne peut pas se désolidariser du dispositif de pose et de dépose. Les moyens de fixation ainsi formés sont faciles à contrôler et ils sont très fiables. Selon un premier mode de réalisation préférentiel, les moyens de retenue comportent une excroissance apte à venir se loger dans la partie complémentaire de la prothèse qui est alors formée par un logement de la prothèse.

Selon un deuxième mode de réalisation, on pourrait également envisager que les moyens de retenue comportent une cavité dans laquelle vient se loger la partie complémentaire de la prothèse formée par une protubérance de la prothèse. Avantageusement, les moyens de blocage sont formés par une bague, qui est agencée de manière à empêcher le mouvement de la dent dans la direction de libération lorsqu'elle est en position fermée.

Selon différents modes de réalisation :
- Les moyens de fixation peuvent venir autour d'une partie de la prothèse sur laquelle ils viennent se fixer, ou alors
- Les moyens de fixation peuvent venir à l'intérieur d'une partie de la prothèse sur laquelle ils viennent se fixer.

Dans le premier cas, les moyens de retenue viennent de préférence se fixer sur une partie extérieure de la prothèse et la bague est de préférence conformée de façon à ce que, lorsqu'elle est en position fermée, elle entoure une partie de la prothèse assemblée avec les moyens de retenue. Dans ce cas, la zone d'assemblage est de préférence formée par un logement de fixation apte à recevoir une extrémité de la prothèse.

Dans le deuxième cas, les moyens de retenue viennent de préférence se fixer sur une partie intérieure de la prothèse et la bague est de préférence conformée de façon à ce que, lorsqu'elle est en position fermée, elle soit entourée par une partie de la prothèse assemblée avec les moyens de retenue. Dans ce cas, la zone d'assemblage est de préférence formée par un tenon apte à venir s'insérer dans une mortaise de la prothèse.

Le dispositif de pose et de dépose s'étend de préférence autour d'une ligne de référence qui est au centre du dispositif de pose et de dépose. La ligne de référence est de préférence courbée, puisque le dispositif de pose et de dépose est de préférence courbé au repos. Par ailleurs, la ligne de référence suit les mouvements du dispositif de pose et de dépose lorsque celui-ci se déforme.

Selon un mode de réalisation préférentiel, les moyens de blocage peuvent se déplacer longitudinalement pour passer de la position fermée à la position ouverte, les moyens de contrôle comportant un curseur qui peut se déplacer longitudinalement, les moyens de liaison comportant un tube de liaison qui relie le curseur aux moyens de blocage de façon à déplacer longitudinalement les moyens de blocage lorsque le curseur se déplace longitudinalement. Un tel dispositif de pose et de dépose est simple à réaliser, peu couteux et fiable. En outre, un tel dispositif de pose et de dépose est compact.

Toutefois, on pourrait également envisager un dispositif de pose et de dépose dans lequel le curseur et les moyens de blocage peuvent se déplacer en rotation autour de la ligne de référence, ou selon un mouvement hélicoïdal autour de la ligne de référence pour passer de la position fermée à la position ouverte. Dans ce cas, les moyens de liaison comporteraient de préférence également un tube de liaison qui relie le curseur aux moyens de blocage de façon à déplacer en rotation, respectivement suivant un mouvement hélicoïdal, les moyens de blocage lorsque le curseur se déplace en rotation, respectivement suivant un mouvement hélicoïdal. Avantageusement, le dispositif de pose et de dépose est en outre pourvu de moyens de guidage dans lesquels un guide filaire peut être inséré. Ces moyens de guidage permettent de faciliter la mise en place de la prothèse dans le corps du patient et ils permettent un positionnement plus précis de la prothèse. En outre, ils permettent de rigidifier les moyens de liaison de façon à faciliter l'insertion du dispositif de pose et de dépose dans le corps du patient.

Avantageusement, les moyens de guidage traversent le dispositif de pose et de dépose de part en part, ce qui permet de faciliter le guidage du dispositif de pose et de dépose en le faisant coulisser sur le guide filaire qui traverse le dispositif de pose et de dépose de part en part.

En outre, les moyens de guidage sont de préférence disposés au centre du dispositif de pose et de dépose. Ainsi, le tube de liaison entoure de préférence le tube de guidage. De même, les moyens de préhension entourent de préférence les moyens de guidage, tout comme les moyens de fixation entourent de préférence les moyens de guidage.

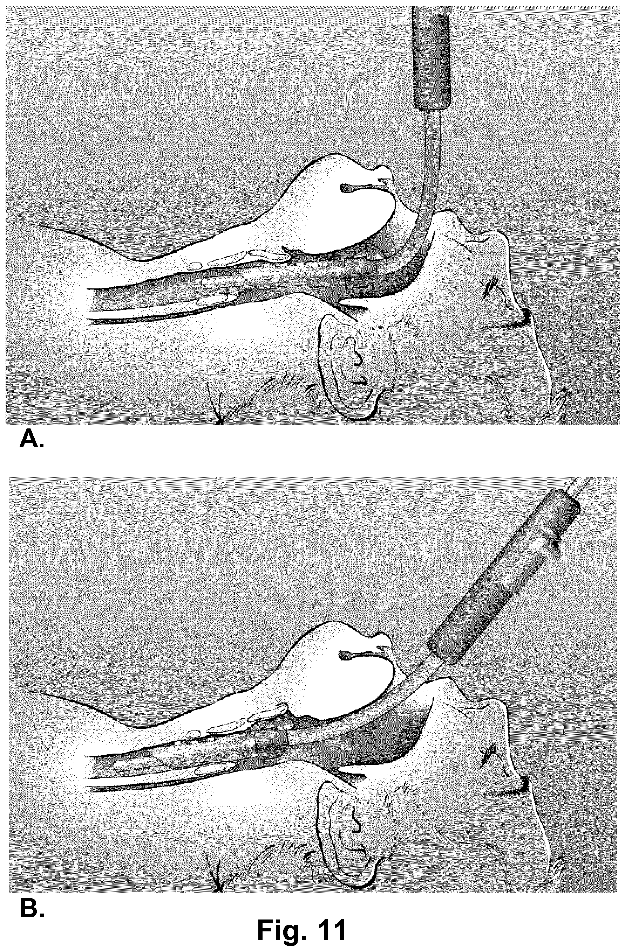

Les moyens de liaison et les moyens de guidage forment un tube flexible entre les moyens de préhension et les moyens de fixation, ce qui leur permet d'être compacts, et ce qui facilite l'insertion du dispositif de pose et de dépose dans le corps du patient (Figure 11). Le tube flexible est ainsi préférentiellement formé par deux tubes concentriques : le tube de liaison et le tube de guidage. Le tube de liaison peut se déplacer en translation par rapport au tube de guidage pour déplacer en translation les moyens de blocage. Le tube de liaison peut donc coulisser par rapport au tube de guidage.

Ce tube flexible présente de préférence une longueur adaptée en fonction de l'endroit du corps dans lequel la prothèse doit être placée.

Selon un mode de réalisation préférentiel, ce tube flexible présente une courbure au repos d'un angle compris entre 130° et 180°, ce qui permet de faciliter l'insertion du dispositif dans le corps du patient. Le rayon de courbure du tube flexible est de préférence de 140 mm +/- 20mm sur 30° +/- 5°.

Ainsi, de préférence, l'ancillaire possède une courbure permettant :
- que les éléments de contrôle aient une position ergonomique pour l'utilisateur. La manipulation de l'ancillaire n'est pas « gênée » par le visage du patient.
- que les éléments de liaison suivent la courbure anatomique naturelle de la langue (Figure 11)

Dans ce mode de réalisation, la courbure (au repos) des moyens de liaison est définie par un angle de courbure compris entre 130° et 170°. De préférence l'ancillaire décrit possède un rayon de courbure de 140 mm +/- 20mm sur 30° +/- 5°. Il est à noter que la partie courbe des moyens de liaison est destinée à être introduite dans l'organisme du patient (en particulier l'orifice endobuccal), et permet de faire passer la prothèse au-delà de la base de la langue lors de son positionnement.

Il est toutefois possible que l'ancillaire de pose présente un angle de courbure de 180° au repos. Dans ce cas, les moyens de liaison et éventuellement de guidage sont droits au repos.

Dans ce cas, on préfère lorsque l'ancillaire présente des moyens d'induction de courbure permettant d'induire une courbure des moyens de liaison et de guidage. On peut notamment obtenir ce résultat lorsque les moyens de liaison et éventuellement guidage se présentent sous la forme de tronçons solidarisés (immobiles) au repos, et mobiles les uns par rapport aux autres lorsque ces moyens d'induction de courbure sont actionnés. Dans ce mode de réalisation, on obtient ainsi la possibilité de la déformation des moyens de liaison et éventuellement des moyens de guidage si on leur applique une force.

Les éléments liant les moyens de préhension aux moyens de fixation présentent, de préférence, un diamètre extérieur inférieur à 11 mm, de préférence de l'ordre de 10,10 mm +/- 0,1:
- Tube bloqueur (tube de liaison) : diamètre intérieur 8,97 mm +/- 0,1 ; diamètre extérieur 10,10 mm +/- 0,1.
- Tube guide dent (tube de guidage), élément de guidage : diamètre intérieur 6,97 mm +/- 0,1 ; diamètre extérieur 8,10 mm +/- 0,1.
- Guide filaire : diamètre 5,30 mm +0 -0,2.

Le dispositif de pose et de dépose peut également comprendre des moyens de verrouillage permettant de maintenir les moyens de blocage en position fermée lorsqu'ils sont en position fermée, et/ou permettant de maintenir les moyens de blocage en position ouverte lorsqu'ils sont en position ouverte, de façon à éviter les fausses manipulations du chirurgien pendant l'opération. Ces moyens de verrouillage sont de préférence formés par des parties en saillie positionnées sur le curseur et sur le manche de façon à freiner le passage du curseur de la position fermée à la position ouverte et inversement. Il y a un « cran » à passer pour chacune des positions ouverte et fermée.

Le dispositif de pose et de dépose permet de mettre en place une prothèse intra laryngée dans le larynx d'un patient. Dans ce cas, le tube flexible présente de préférence une longueur comprise entre 10 et 15 cm, ce qui permet au chirurgien de pouvoir manipuler les moyens de préhension et de contrôle tandis que les moyens de fixation sont dans le larynx du patient. En outre, le tube flexible ne doit pas être trop long de façon à faciliter l'insertion et la manipulation du dispositif de pose et de dépose, muni de la prothèse à son extrémité, dans le corps du patient.

Par ailleurs, le tube flexible doit avoir un minimum de rigidité de façon à pouvoir pousser les moyens de fixation solidaires de la prothèse à travers le corps du patient.

Avantageusement, les moyens de fixation comportent en outre des moyens d'orientation aptes à n'autoriser l'assemblage de la prothèse et de la zone d'assemblage que lorsque la prothèse présente une orientation prédéterminée vis-à-vis de la zone d'assemblage. Ces moyens d'orientation permettent de contrôler à la fois l'orientation de la prothèse dans le dispositif de pose et de dépose pour savoir dans quel sens insérer la prothèse dans le dispositif de pose et de dépose. Ces moyens d'orientation permettent en outre de positionner la prothèse en rotation dans le larynx du patient en faisant tourner le dispositif de pose et de dépose autour de l'axe de référence. Les moyens d'orientation permettent donc de transmettre un couple de rotation du dispositif de pose et de dépose à la prothèse. Si la prothèse n'est pas positionnée correctement en rotation, le chirurgien doit la sortir du larynx du patient de façon à la réintroduire dans la bonne position angulaire. En effet, la prothèse peut présenter sur sa surface extérieure des protubérances qui pourraient blesser le patient si la prothèse était tournée à l'intérieur du corps du patient.

Les moyens d'orientation sont de préférence disposés sur une paroi de la zone d'assemblage. Par exemple, lorsque la zone d'assemblage est un logement de fixation dans laquelle vient s'insérer la prothèse, les moyens d'orientation sont de préférence formés par une ouverture dans une paroi du logement de fixation, dans laquelle une protubérance de la prothèse peut s'insérer. Cette ouverture permet en outre au dispositif de pose et de dépose de se séparer plus facilement de la prothèse lors du retrait du dispositif de pose par le chirurgien.

Le dispositif de pose et de dépose est réalisé dans des matériaux biocompatibles, au moins à court terme. Ainsi, chacun des tubes de liaison et de guidage est de préférence réalisé en polyéthylène. Les autres pièces du dispositif de pose et de dépose sont de préférence réalisées en polyoxyméthylène, ou autre ainsi que vu plus haut pour les moyens de fixation.

Un deuxième aspect de l'invention concerne également un kit de pose et de dépose d'une prothèse comportant :
- Un dispositif de pose et de dépose selon le premier aspect de l'invention ;
- Un guide filaire.

Le guide filaire est de préférence réalisé en PVC.

Selon différents modes de réalisation, le guide filaire peut être droit ou alors il peut être courbé, notamment à au moins une de ses extrémités de façon à optimiser sa fonction de guidage dans le corps du patient. Le guide filaire pourrait notamment comporter une portion angulée à une de ses extrémités. Cette portion recourbée se situe de préférence à proximité d'une extrémité du guide.

Ce kit est de préférence à usage unique. Toutefois, on pourrait également envisager de pouvoir le stériliser plusieurs fois, et dans ce cas, il pourrait être ré-utilisable. Dans un mode de réalisation préféré, ledit kit comporte aussi une prothèse, de préférence une prothèse laryngée ou intra-laryngée, en particulier telle que décrite plus loin.

Un autre exemple se rapporte à un kit comportant un dispositif de pose et de dépose selon le premier aspect de l'invention, ainsi qu'une prothèse laryngée ou intra-laryngée, en particulier telle que décrite plus loin.

Le dispositif de pose et de dépose est un dispositif de pose et de dépose d'une prothèse intralaryngée.

L'invention décrit ainsi ce dispositif de pose et dépose pour sa mise en oeuvre et son utilisation pour la pose et/ou la dépose d'une prothèse intralaryngée par voie buccale ou endobuccale. En particulier, cette pose et/ou dépose de la prothèse est effectuée sur un patient n'ayant pas subi de trachéotomie. On peut toutefois effectuer cette pose chez des patients ayant subi une trachéotomie. En utilisant l'ancillaire selon l'invention, il n'est alors plus besoin de « tirer » la prothèse par le trou de trachéotomie.

Ainsi, l'ancillaire (et éventuellement le guide filaire) possède préférentiellement une lumière centrale permettant le passage de l'air de l'extérieur vers les voies aériennes et des voies aériennes vers l'extérieur. Ainsi, de façon préférée, les moyens de guidage et de liaison présentent un orifice central.

Le patient peut donc respirer à travers l'ancillaire, pendant l'utilisation de l'ancillaire.

Est également décrite une méthode de pose d'une prothèse comportant une étape d'introduction de la prothèse dans le corps d'un patient par voie endobuccale à l'aide d'un dispositif de pose et de dépose selon l'invention.

La méthode de pose comporte de préférence une ou plusieurs des étapes suivantes :
- Mise en position ouverte des moyens de fixation du dispositif de pose et de dépose ;
- Assemblage de la prothèse et des moyens de fixation du dispositif de pose et de dépose ;
- Mise en position fermée des moyens de fixation du dispositif de pose et de dépose de façon à ce que les moyens de fixation et la prothèse soient solidaires ;
- Insertion du guide filaire au travers du dispositif de pose et de dépose;
- Insertion dans le corps du patient de la prothèse par voie endobuccale à l'aide du dispositif de pose et de dépose ;
- Positionnement de la prothèse dans une position finale ;
- Retrait du guide filaire ;
- Mise en position ouverte des moyens de fixation du dispositif de pose et de dépose ;
- Libération de la prothèse en retirant le dispositif de pose et de dépose en direction de l'extérieur du corps du patient, de préférence par un petit mouvement sec ;
- Retrait du dispositif de pose et de dépose.

La prothèse insérée est une prothèse intra laryngée. Elle est insérée par voie endobuccale jusqu'à l'espace intra laryngé dans lequel elle est mise en place.

Le dispositif de pose et de dépose permet d'insérer la prothèse dans le larynx du patient en la poussant à travers la voie endobuccale, au lieu de l'insérer en la tirant depuis un trou de trachéotomie.

Si on utilise l'ancillaire de telle sorte que le plant de symétrie de l'ancillaire soit dans le plan sagittal (plan médian) du patient, le positionnement angulaire de la prothèse sera alors optimal (c'est-à-dire que la plan de symétrie de la prothèse sera également dans le plan sagittal du patient).

L'enfoncement de la prothèse dans la gorge du patient peut être contrôlé par endoscopie. On verra aussi ci-dessous la façon de déterminer le bon positionnement de la prothèse en fonction du type de prothèse utilisée.

Est également décrite une méthode de dépose d'une prothèse disposée dans le corps d'un patient par voie buccale à l'aide d'un dispositif de pose et de dépose selon le premier aspect de l'invention.

La méthode de dépose comporte de préférence une ou plusieurs des étapes suivantes :
- Mise en position ouverte des moyens de fixation du dispositif de pose et de dépose ;
- Insertion du dispositif de pose et de dépose par voie endobuccale jusqu'à ce qu'il atteigne la prothèse et que les moyens de fixation s'assemblent avec la prothèse ;
- Mise en position fermée des moyens de fixation de façon à ce que la prothèse soit solidaire des moyens de fixation ;
- Retrait de la prothèse du corps du patient à l'aide du dispositif de pose et de dépose.

La prothèse qui est déposée ou retirée du corps du patient est une prothèse intra laryngée ou laryngée qui était mise en place dans l'espace intra laryngé ou en remplacement du larynx d'un patient.

Dans un mode de réalisation, ladite prothèse laryngée ou intra-laryngée peut être telle que décrite dans WO 2013/034858. Cette prothèse peut ainsi être un dispositif à clapets, comportant une portion distale formant armature porteuse annulaire et une portion centrale formant obturateur destiné à permettre le passage de l'air et à empêcher de manière hermétique le passage de tout autre élément, l'obturateur comprenant i) une partie périphérique formant un premier clapet solidaire de l'armature porteuse annulaire au niveau d'une première région charnière, et ii) une partie centrale formant un second clapet solidaire du premier clapet au niveau d'une seconde région charnière (ces clapets sont décrits plus bas). Ce dispositif comprend également un moyen formant collerette ou jupe situé en-dessous de l'armature porteuse annulaire. Dans un mode de réalisation particulier, l'armature porteuse annulaire ainsi que les deux clapets et formant obturateur sont en métal massif (notamment le titane). De préférence, ce moyen formant collerette ou jupe situé en-dessous de l'armature porteuse annulaire est en silicone. Par ailleurs, ce moyen formant jupe est préférentiellement constitué d'une première partie située en haut du dispositif et fixée à l'armature porteuse annulaire, d'une seconde partie dont le diamètre est plus faible que la première partie et située en-dessous de celle-ci, et enfin d'une troisième partie dont le diamètre est supérieur à celui de la seconde partie et située en-dessous de la seconde partie et qui comporte une découpe en biseau. On préfère lorsque la surface extérieure de la jupe est munie d'ergots et/ou lorsque la surface intérieure de la jupe comporte une surépaisseur plane sur toute sa hauteur, afin d'augmenter la rigidité du dispositif. Les ergots sont destinés à bloquer la prothèse en translation et en rotation une fois que celle-ci est mise en place au sein du larynx du patient. Une fois positionnée, cette prothèse est bloquée par le cartilage cricoïde, au niveau de la seconde partie de la jupe (Figure 11.B). De préférence, le diamètre intérieur de la jupe permet le passage d'une canule de 6,0 mm de diamètre afin que d'éventuels examens médicaux puissent être réalisés. La majorité des arêtes de la jupe (9) est arrondie afin d'être atraumatique d'une part, et de faciliter l'évacuation des mucosités des voies respiratoires inférieures vers la zone pharyngée d' autre part.

Une autre prothèse intra-laryngée utilisable est décrite dans EP 815807.

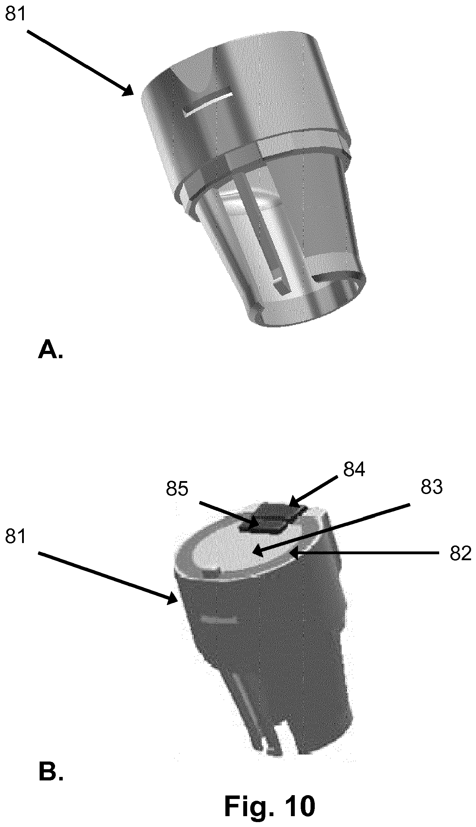

Dans un autre mode de réalisation, ladite prothèse laryngée ou intra-laryngée (Figure 10) est avantageusement composée d'un corps (81) en métal biocompatible (préférentiellement en titane massif). En partie supérieure de cette prothèse (qui se situera de façon proximale à la base de la langue) se situent deux clapets concentriques (82, 83). L'ouverture du petit clapet (83) vers le bas permet l'inspiration, l'ouverture des deux clapets (petit + grand) vers le haut permet l'expiration.

Dans un mode de réalisation, la prothèse laryngée ou intralaryngée présente une portion distale (81) formant armature porteuse annulaire (et dont une partie est destinée à être fixée à un dispositif qui a été pré-positionné et fixé à la limite de la trachée du patient, tel que décrit dans WO 2013/079362), et une portion centrale formant obturateur destiné à permettre le passage de l'air et à empêcher de manière hermétique le passage de tout autre élément.

Dans un mode de réalisation préféré, ledit obturateur comprend i) une partie périphérique formant un premier clapet (82) solidaire de l'armature porteuse annulaire au niveau d'une première région charnière (84), ledit premier clapet (82) étant capable de se soulever sous l'effet de la surpression résultant de l'expiration du patient et ii) une partie centrale formant un second clapet (83) solidaire du premier clapet (82) au niveau d'une seconde région charnière (85), ledit second clapet étant capable non seulement de s'abaisser sous l'effet de la dépression exercée par l'air inspiré par le patient mais également de coopérer avec le premier clapet pour se relever suite à l'expiration du patient, lesdits premier et second clapets coopérant entre eux de manière totalement hermétique.

Dans un mode de réalisation préféré, les moyens d'orientation présents dans les moyens de fixation du dispositif selon l'invention permettent d'orienter la prothèse par rapport à la zone faisant charnière pour les deux clapets de ladite prothèse tels que décrits plus haut. De fait, cette zone charnière doit être appliquée contre la base de langue du patient et il est donc important que le chirurgien connaisse l'orientation de la prothèse lors de l'introduction, ce qui est rendu possible du fait de la présence de ces moyens d'orientation.

On peut aussi noter que les charnières (84) et (85) sont préférentiellement réalisées dans un matériau semi-rigide ou encore rigide. En effet, il convient à la fois que le mouvement soit rendu possible avec une faible pression respiratoire mais également que les charnières permettent aux clapets de résister à la pression du bol alimentaire ou de tout fluide qui viendrait appuyer sur l'obturateur avec plus ou moins de force. Par ailleurs, il faut que les charnières résistent à la colonisation et assurent un mouvement régulier et pérenne des clapets. Enfin, la zone charnière doit assurer une parfaite stabilité des axes de rotation par rapport à l'armature porteuse du dispositif afin de garantir l'étanchéité à la fermeture.

Dans le mode de réalisation préféré, un tel matériau semi-rigide (suffisamment souple pour pouvoir assurer une certaine élasticité tout en étant assez rigide pour pouvoir résister et rester en forme sous l'effet d'une faible pression) est sélectionné parmi le plastique, la gomme, une résine ou encore le silicone. Un matériau super élastique comme le nitinol peut également être envisagé. On préfère le silicone, en particulier le silicone 70 Shore A.

La prothèse laryngée ou intra-laryngé peut également présenter également un dispositif d'assistance placé sur l'armature porteuse annulaire (81) et/ou le premier clapet (82). Ce dispositif d'assistance peut être mécanique, électrique ou électronique, mais est préférentiellement un dispositif aimanté. Par dispositif aimanté ou élément aimanté, on entend un ou plusieurs aimants permanents de type lanthanides qui sont biocompatibles ou rendus biocompatibles par différents traitements connus de l'homme du métier, ou encore enfermés hermétiquement dans un logement adapté prévu à cet effet. Ledit dispositif aimanté peut être placé soit sur le premier clapet (82), soit sur la surface interne de l'armature porteuse (81). En vis-à-vis du ou des aimants quand le premier clapet est en position fermée, on place un élément métallique (soit un ou plusieurs éléments métalliques susceptibles d'être aimantés sont placés de façon à entrer en contact avec le ou les aimants quand le premier clapet est en position fermée).

Ainsi, dans un mode de réalisation particulier, la prothèse laryngée ou intra-laryngée comporte un dispositif d'assistance consistant en un élément métallique disposé au niveau de l'armature porteuse annulaire (81) venant en contact avec un élément aimanté disposé au niveau du premier clapet (82). Dans un mode de réalisation préféré, la prothèse laryngée comportant un dispositif d'assistance consistant en un élément métallique disposé au niveau du premier clapet (82) venant en contact avec un élément aimanté disposé au niveau de l'armature porteuse annulaire (81).

Un dispositif d'assistance mécanique peut aussi être positionné sur chacun des clapets afin de faciliter leur coopération dans le fonctionnement souhaité. Selon ce mode de réalisation la prothèse laryngée est telle que le premier clapet (82) et le second clapet (83) comprennent un dispositif d'assistance de manière à permettre le soulèvement uniquement du second clapet (83) et l'abaissement simultané du premier clapet (82) et du second clapet (83). Dans un mode de réalisation préféré, les clapets (82, 83) du dispositif selon l'invention comprennent un dispositif d'assistance de manière à permettre l'abaissement uniquement du second clapet (83) et le soulèvement simultané du premier clapet (82) et du second clapet (83).

### BREVES DESCRIPTION DES FIGURES

D'autres caractéristiques et avantages de l'invention ressortiront à la lecture de la description détaillée qui suit, en référence aux figures annexées, qui illustrent :
- La figure 1, une vue en coupe d'un dispositif de pose et de dépose selon un mode de réalisation de l'invention ;
- La figure 2, une vue en coupe du dispositif de la figure 1 dans lequel est insérée une prothèse intra laryngée et un guide filaire;
- La figure 3, une vue en coupe des moyens de fixations du dispositif de pose et de dépose de la figure 1 lorsqu'ils sont en position ouverte et que la prothèse intra laryngée n'est pas encore insérée dans ces moyens de fixation ;
- La figure 4, une vue en coupe des moyens de fixation de la figure 3, lorsqu'ils sont en position ouverte et que la prothèse intra laryngée est insérée dans les moyens de fixation ;
- La figure 5, une vue en coupe des moyens de fixation de la figure 4, lorsqu'ils sont en position fermée sur la prothèse intra laryngée ;
- La figure 6, une vue en perspective d'un kit de pose comportant le dispositif de la figure 1 lorsqu'il est en position ouverte ;
- La figure 7, une vue en perspective du dispositif de la figure 1 lorsqu'il est en position ouverte et qu'une prothèse intra laryngée est insérée dans ce dispositif ;
- La figure 8, une vue en perspective du dispositif de la figure 1 lorsqu'il est en position fermée et qu'une prothèse intra laryngée lui est attachée ;
- La figure 9, une vue en coupe du manche du dispositif de la figure 1.
- La figure 10, une prothèse laryngée (81) qui présente les éléments nécessaires pour obtenir une fonction similaire au larynx naturel. Cette prothèse est notamment destinée à être fixée sur un dispositif tel que décrit dans WO 2013/079362.
- La figure 11, une description de la pose d'une prothèse intralaryngée par voie endobuccale. 11.A: introduction de la prothèse. 11.B : positionnement de la prothèse. On observe donc la déformation des moyens de liaison flexible du dispositif lors de l'introduction de la prothèse. Cette prothèse est similaire à celle décrite dans WO 2013/034858.

Pour plus de clarté, les éléments identiques ou similaires sont repérés par des signes de références identiques sur l'ensemble des figures.

### DESCRIPTION DETAILLEE D'AU MOINS UN MODE DE REALISATION

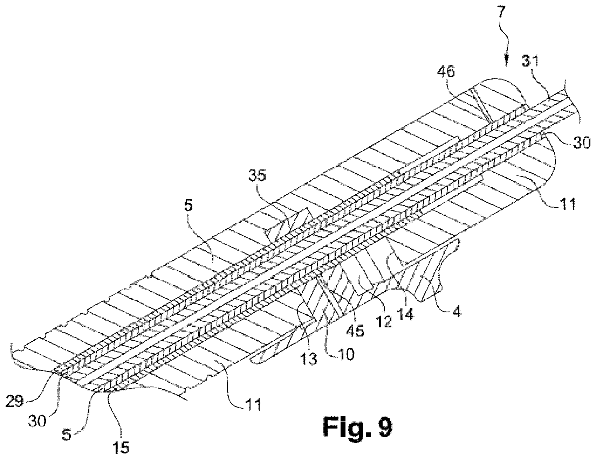

En référence aux figure 1 à 9, un dispositif de pose et de dépose 1 selon un mode de réalisation de l'invention va maintenant être décrit plus en détail. Ce dispositif de pose et de dépose permet la mise en place d'une prothèse intra laryngée 40. Toutefois, dans un exemple ne formant pas partie de l'invention, il pourrait également permettre la mise en place d'autres types de prothèses.

Le dispositif de pose et de dépose 1 s'étend suivant une ligne de référence 6. Cette ligne de référence 6 est courbe puisque le dispositif 1 présente une courbure d'un rayon de courbure de préférence de 140 mm +/- 20mm sur 30° +/- 5°. La courbure de la ligne de référence peut être modifiée lorsque le dispositif est plié dans un sens ou dans l'autre. Dans ce document, on qualifie de « longitudinal » ou « axial » un élément qui s'étend sensiblement suivant la ligne de référence, et on qualifie de « transversal », un élément qui s'étend sensiblement perpendiculairement à l'axe de référence 6.

Le dispositif de pose et de dépose 1 comporte une extrémité proximale 7 et une extrémité distale 8. Le dispositif de pose et de dépose 1 comporte :
- Des moyens de préhension 3 situés au niveau de l'extrémité proximale 7 ;
- Des moyens de contrôle 4 situés sur les moyens de préhension 3 ;
- des moyens de fixation 2 situés au niveau de l'extrémité distale 8 ;
- des moyens de liaison 5 qui relient les moyens de contrôle 4 aux moyens de fixation 2 ;
- des moyens de guidage 15.

Les moyens de préhension 3 sont formés dans cet exemple de réalisation par un manche 9 qui s'étend suivant l'axe longitudinal 6. Le manche 9 présente dans cet exemple une forme sensiblement cylindrique autour de l'axe de référence 6. Le manche 9 est réalisé en polyoxyméthylène dans cet exemple de réalisation.

Les moyens de contrôle 4 sont formés par un curseur 10. Le curseur 10 est situé sur une surface latérale 11 du manche 9. Le curseur 10 est inséré dans un logement 12 du manche 9, dans lequel le curseur 10 peut être déplacé longitudinalement entre une position fermée dans laquelle il est en butée contre la paroi transversale 13 du logement 12 (cas représenté sur la figure 1), et une position ouverte dans laquelle il est en butée contre la paroi transversale 14 du logement 12. Le curseur 10 est réalisé en polyoxyméthylène dans cet exemple.

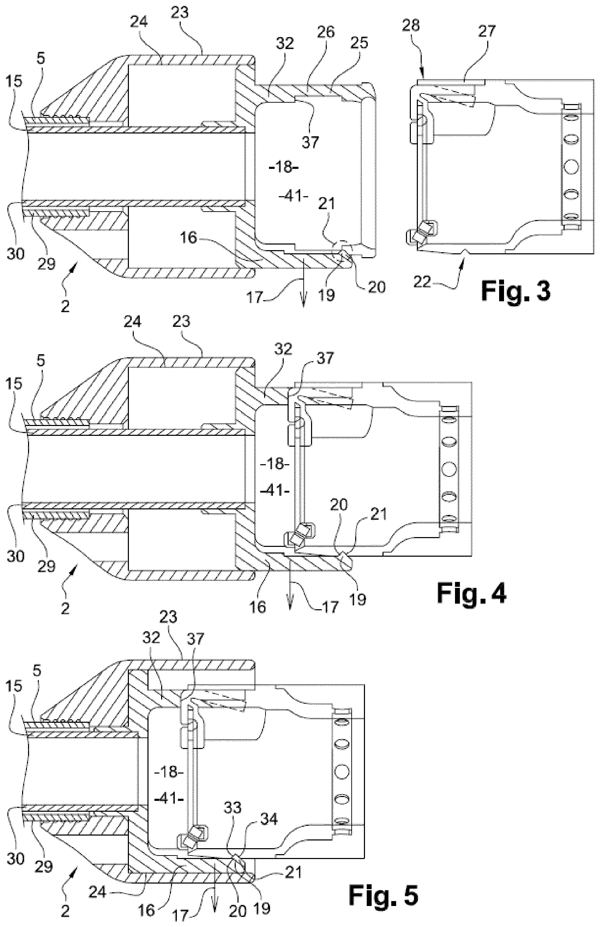

Les moyens de fixation 2 sont représentés plus précisément sur les figures 3 à 5. Les moyens de fixation 2 comportent une zone d'assemblage 41 qui est ici formée par un logement de fixation 18 dans lequel une extrémité 28 d'une prothèse peut être insérée.

Les moyens de fixation 2 comportent des moyens d'orientation 25 qui permettent d'insérer la prothèse dans le logement de fixation 18 dans une seule position. Ces moyens d'orientation 25 sont formés dans ce mode de réalisation par une ouverture 26 réalisée sur une partie de la circonférence de la paroi latérale 32 du logement de fixation 18. La prothèse 40 comporte sur son extrémité 28 qui doit venir s'insérer dans le logement de fixation 18 une protubérance 27 dimensionnée pour pouvoir s'insérer dans l'ouverture 26. Les dimensions transversales du logement de fixation 18 sont telles que l'extrémité 28 de la prothèse 40 ne puisse pas s'insérer dans le logement de fixation 18 dans une autre position que celle dans laquelle la protubérance 27 est insérée dans l'ouverture 26.

Les moyens de fixation 2 comportent également une dent 16. La dent est de préférence formée sur une partie de la circonférence du logement de fixation 18. La dent 16 est de préférence positionnée de façon à être diamétralement opposée à l'ouverture 26. La dent 16 est en saillie axiale sur la paroi latérale 32 du logement de fixation 18. La dent 16 est agencée de façon à pouvoir se déformer selon une direction 17, appelée « direction de libération ». La direction de libération 17 est de préférence sensiblement perpendiculaire à l'axe de référence 6. Pour cela, le logement de fixation 18 et la dent 16 sont de préférence formés en polyoxyméthylène. La dent 16 peut par exemple présenter une section rectangulaire de 2,6 mm par 1,6 mm, et une longueur, depuis le bord du logement de fixation 18, de 10 mm.

La dent 16 présente une extrémité distale 19 pourvue de moyens de retenue 20. Ces moyens de retenue 20 sont ici formés par une excroissance 21 qui fait saillie transversalement de la dent 16 en direction de l'intérieur du logement de fixation 18. L'excroissance 21 est destinée à venir s'insérer dans un logement 22 de la prothèse 40. L'excroissance 21 présente de préférence une section triangulaire formée par deux parois 33, 34 inclinées par rapport à l'axe de référence, et formant entre elles une arête arrondie, ce qui permet de faciliter la sortie de l'excroissance 21 du logement 22 de la prothèse. Une telle excroissance 21 peut s'insérer et sortir facilement du logement 22 lorsque le dispositif de pose et de dépose se translate par rapport à la prothèse grâce à la présence des parois inclinées 33, 34.

Les moyens de fixation 2 comportent également des moyens de blocage 23. Les moyens de blocage 23 sont ici formés par une bague 24 qui peut être translatée longitudinalement suivant l'axe de référence 6 entre une position fermée, représentée sur la figure 5, dans laquelle elle longe la dent 16 jusqu'à son extrémité distale 19 et une position ouverte, représentée sur la figure 3, dans laquelle elle est en retrait de la zone d'assemblage 41. La bague 24 présente des dimensions transversales telles que, lorsque la bague 24 est dans la position fermée, elle longe la dent 16 sur toute sa longueur de façon à empêcher les mouvements de la dent 16 dans la direction de libération 17.

Les moyens de blocage 23 sont reliés aux moyens de contrôle 4, et plus précisément au curseur 10, par l'intermédiaire des moyens de liaison 5. Ces moyens de liaison 5 permettent de translater longitudinalement les moyens de blocage 23 lorsque le curseur 10 se translate longitudinalement. Plus précisément, les moyens de liaison 5 permettent de placer la bague 24 dans la position fermée lorsque le curseur 10 est dans la position fermée, et ils permettent de placer la bague 24 dans la position ouverte lorsque le curseur 10 est dans la position ouverte. Pour cela, les moyens de liaison 5 comportent de préférence un tube de liaison 29 qui présente une extrémité proximale 35 reliée au curseur 10 et une extrémité distale 36 reliée à la bague 24. Le tube de liaison 29 est de préférence réalisé en polyéthylène. L'extrémité proximale 35 du tube de liaison 29 est de préférence collée au curseur 10 grâce à au moins une zone de colle 45. De même, l'extrémité du tube de guidage 30 est de préférence fixée au manche 9 par une zone de collage 46. L'extrémité distale 36 du tube de liaison est de préférence collée à la bague 24.

Le dispositif de pose et de dépose 1 comporte également des moyens de guidage 15 dans lesquels un guide filaire peut être inséré. Les moyens de guidage 15 sont de préférence formés par un tube de guidage 30 dans lequel un guide filaire peut être inséré. Le tube de guidage 30 est de préférence agencé de façon à pouvoir également accueillir, outre le guide filaire, un dispositif optique permettant de visualiser la pose de la prothèse. Le tube de guidage 30 est de préférence réalisé en polyéthylène. Ce tube de guidage 30 traverse de préférence de part en part le dispositif de pose et de dépose, depuis son extrémité proximale 7 jusqu'à son extrémité distale 8. Le tube de guidage 30 débouche sur une ouverture transversale au niveau de l'extrémité proximale 7 du dispositif de pose et de dépose appelée « lumière proximale » 38. Le tube de guidage 30 débouche sur une ouverture transversale au niveau de l'extrémité distale appelée « lumière distale » 39. Le tube de guidage 30 est de préférence entouré, entre les moyens de préhension 3 et les moyens de fixation 2 par le tube de liaison 29, de sorte que sur cette portion, le tube de guidage 30 et le tube de liaison 29 forment un tube flexible qui peut se déformer pour s'adapter aux conduits dans lesquels il est inséré. Toutefois, ce tube flexible doit conserver un minimum de rigidité pour pouvoir insérer les moyens de fixation à travers le corps du patient.

Ainsi, on préfère lorsque les matériaux utilisés pour les moyens de liaison et pour le guide filaire ont des propriétés mécaniques qui permettent :
- leur déformation pour s'adapter aux différentes anatomies des patients.
- leur résistance mécanique pour transmettre l'effort nécessaire à la mise en place de la prothèse (ici dans le larynx).

Il est donc particulièrement adapté de choisir, en tant que matériau utilisé pour les éléments de liaison, du polyéthylène, et notamment des combinaisons de polyéthylène haute densité et basse densité (PEHD/PEBD).

Le guide filaire (31) doit être plus flexible et l'on utilise ainsi notamment le PVC.

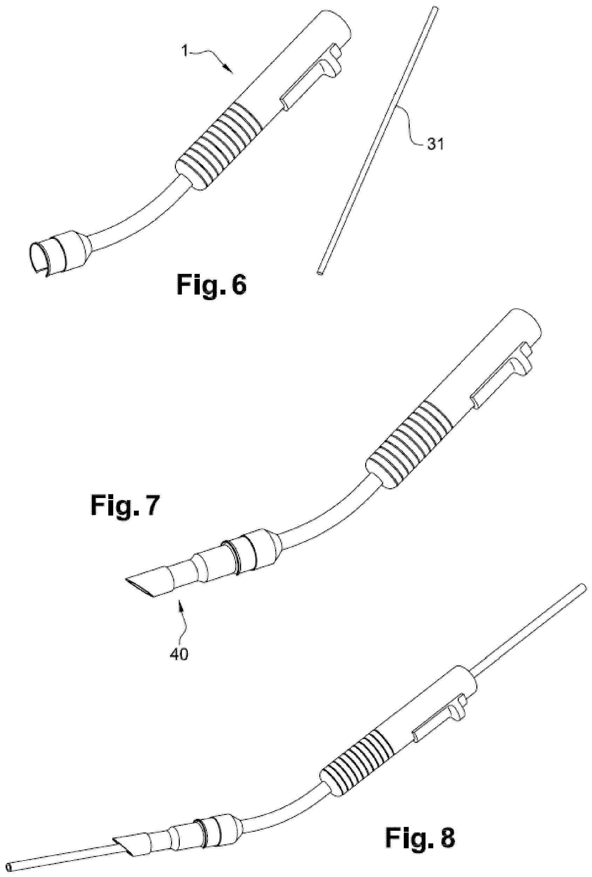

La figure 6 représente un kit de pose et de dépose comportant le dispositif de pose et de dépose décrit précédemment, ainsi qu'un guide filaire 31. Le guide filaire 31 est de préférence cylindrique. Il est de préférence en polyéthylène. Il peut par exemple présenter un diamètre 5,3 mm de diamètre, lorsque le tube de guidage 30 présente un diamètre de 6,97 mm de diamètre.

Le fonctionnement du dispositif de pose et de dépose décrit en référence aux figures 1 à 5 va maintenant être décrit en référence aux figures 6 à 8.

Dans un premier temps, les moyens de fixation sont placés en position ouverte, comme représenté sur la figure 6. Pour cela, le curseur 10 est translaté en direction de l'extrémité proximale 7 du dispositif de pose et de dépose de façon à ce qu'il vienne en butée contre la paroi transversale 14. Cette translation du curseur 10 entraine la translation des moyens de blocage 23 via les moyens de liaison 5. Les moyens de blocage 23 sont alors dans la position ouverte représentée sur la figure 5, dans laquelle il n'empêchent pas le mouvement de la dent 16 dans la direction de libération 17.

La prothèse 40 est alors insérée dans les moyens de fixation 2 et plus précisément dans le logement de fixation 18, comme représenté sur la figure 7, jusqu'à ce que la prothèse vienne en butée contre une butée 37 du logement de fixation 18. Une seule orientation de la prothèse dans le logement de fixation 18 est possible grâce aux moyens d'orientation 25. En effet, la prothèse ne peut pénétrer dans le logement de fixation 18 que lorsque la protubérance 27 de la prothèse est en face de l'ouverture 26 du dispositif de pose et de dépose. Lors de l'insertion de l'extrémité 28 de la prothèse dans le logement de fixation 18, les moyens de retenue 20 de la dent 16 pénètrent dans le logement 22 de la prothèse suite à la déformation dans la direction de libération 17 de la dent 16. L'extrémité 28 de la prothèse présente de préférence une rampe qui permet de faciliter l'insertion de la prothèse dans la zone d'assemblage malgré la présence de la dent pourvue des moyens de retenue. Cette rampe permet d'amorcer la déformation de la dent dans la direction de libération de façon à ce que l'extrémité de la prothèse pénètre dans le logement de fixation 18.

Le curseur 10 est alors translaté suivant l'axe de référence 6 en direction de l'extrémité distale 8 du dispositif de pose et de dépose jusqu'à ce qu'il vienne en butée contre la paroi transversale 13. Cette translation du curseur 10 entraine la translation des moyens de blocage 23 en direction de l'extrémité distale 8 du dispositif de pose et de dépose via les moyens de liaison 5. Les moyens de blocage 23 passent alors en position fermée puisqu'ils longent la dent 16 de façon à empêcher ses mouvements selon la direction de libération. Les moyens de fixation 2 sont alors en position fermée de sorte qu'ils emprisonnent la prothèse.

Le guide filaire 31 est alors inséré dans le tube de guidage 30 par la lumière proximale 38 jusqu'à ce qu'il dépasse de la lumière distale 39 d'environ 10 cm comme représenté sur la figure 8.

La prothèse est ensuite poussée à l'aide du dispositif de pose et de dépose jusqu'à ce qu'elle s'impacte dans le cartilage cricoïde. L'utilisation du guide filaire 31 permet de sécuriser le trajet de la prothèse et de son système de pose jusqu'au lieu d'implantation : on évite ainsi par exemple de prendre la direction de l'oesophage. Les moyens d'orientation 25 permettent de positionner en rotation la prothèse à l'intérieur de du larynx puisqu'ils permettent de tourner la prothèse lorsque le manche 9 est tourné. L'orientation de la prothèse peut donc être contrôlée facilement depuis l'extérieur par un chirurgien grâce au manche 9. En cas de mauvais positionnement de la prothèse, il est plus simple de ressortir la prothèse du larynx et de la réimpacter dans le cartilage cricoïde au lieu de la tourner directement dans le corps du patient. On évite ainsi de traumatiser les tissus du larynx avec les picots en silicone de la prothèse dont le rôle est justement d'éviter toute rotation de la prothèse une fois implantée.

Une fois la prothèse mise en place, le guide filaire 31 est retiré, puis les moyens de fixation sont à nouveau placés en position ouverte. Pour cela, le curseur 10 est translaté en direction de l'extrémité proximale 7 du dispositif de pose et de dépose, ce qui entraîne la translation des moyens de blocage 23 qui n'empêchent alors plus le mouvement de la dent 16 dans la direction de libération 17. La prothèse est alors libérée du dispositif de pose et de dépose en exerçant un petit mouvement sec de retrait du dispositif de pose et de dépose. La prothèse, qui est freinée par la cavité dans laquelle elle est engagée, reste alors en place, et les moyens de retenues 20, qui présentent une paroi inclinée 33, sortent facilement du logement 22 de la prothèse suite à la déformation dans la direction de libération de la dent 16. L'ouverture des moyens de fixation peut donc être contrôlée facilement depuis l'extérieur du corps du patient par un chirurgien grâce aux moyens de contrôle 4. Après désolidarisation de la prothèse et du dispositif de pose et de dépose, ce dernier peut être retiré.

La dépose de la prothèse par le dispositif de pose et de dépose peut avoir suivant le même principe : le dispositif de pose et de dépose, dont les moyens de fixation sont en position ouverte, est inséré dans l'espace sus glottique jusqu'à ce qu'il atteigne la prothèse. Le dispositif de pose et de dépose est guidé jusqu'à ce que l'extrémité 28 de la prothèse pénètre dans le logement de fixation 18, et plus précisément, jusqu'à ce que l'extrémité 28 de la prothèse vienne en butée contre la butée 37. Lors de l'insertion du logement de fixation 18 autour de l'extrémité 28 de la prothèse, la dent 16 se déforme dans la direction de libération 17 lorsque l'excroissance 21 longe la paroi latérale de l'extrémité 28 de la prothèse, jusqu'à ce que l'excroissance 21 atteigne le logement 22 de la prothèse, dans lequel elle vient se loger. Le curseur 10 est alors translaté en direction de l'extrémité distale 8 de façon à ce qu'ils entraînent les moyens de blocage 23 en position fermée. Les moyens de blocage 23 viennent alors longer la dent 16 sur toute sa longueur de façon à empêcher les déformations de la dent 16 dans la direction de libération. La prothèse est alors solidaire du dispositif de pose et de dépose. L'ensemble formé par la prothèse et le dispositif de pose et de dépose est alors retiré de l'espace sus-glottique. Le guidage du dispositif de pose et de dépose peut être facilité lors de l'opération de dépose par l'utilisation du guide filaire 31.

Naturellement, l'invention n'est pas limitée aux modes de réalisation décrits en référence aux figures et des variantes pourraient être envisagées sans sortir du cadre de l'invention telle que décrite dans les revendications. Ainsi, la commande de la position ouverte à la position fermée, et inversement, des moyens de fixation par les moyens de contrôle pourrait avoir lieu non pas par un mouvement de translation mais par un mouvement de rotation ou par un mouvement hélicoïdal. En outre, les moyens de fixation pourraient être insérés à l'intérieur de l'extrémité 28 de la prothèse, au lieu que ce soit l'extrémité 28 de la prothèse qui soit insérée dans les moyens de fixation. On pourrait également envisager d'utiliser d'autres moyens de retenue, ainsi que d'autres matériaux. En outre, le dispositif de pose et de dépose pourrait permettre la pose et la dépose d'autres types de prothèse, dans un exemple ne formant pas partie de l'invention.

## Revendications

1. Dispositif de pose et de dépose (1) d'une prothèse (40) par voie endobuccale comportant :
- Des moyens de fixation (2) aptes à se fixer sur la prothèse (40), les moyens de fixation (2) pouvant être placés dans deux positions :
∘ Une position fermée dans laquelle ils sont solidaires de la prothèse (40);
∘ Une position ouverte dans laquelle ils ne sont pas solidaires de la prothèse ;
- Des moyens de préhension (3) permettant la manipulation du dispositif de pose et de dépose (1);
- Des moyens de contrôle (4), les moyens de contrôle (4) étant reliés aux moyens de fixation (2), les moyens de contrôle (4) étant aptes à commander les moyens de fixation (2) de façon à les placer dans la position fermée ou dans la position ouverte ;
- Des moyens de liaison (5), au moins en partie flexibles, qui relient les moyens de contrôle (4) aux moyens de fixation (2) de façon à transmettre la commande des moyens de contrôle (4) aux moyens de fixation (2), ces moyens de liaison (5) se déformant lors de l'introduction de la prothèse (40) par voie endobuccale.

2. Dispositif de pose et de dépose (1) selon la revendication 1, dans lequel les moyens de fixation (2) comportent :
- une zone d'assemblage (41) apte à être assemblée avec une partie de la prothèse (40);
- Une dent (16) déformable élastiquement pourvue de moyens de retenue (20) aptes à être retenus par une partie complémentaire (22) de la prothèse (40), la dent (16) devant se déformer élastiquement dans au moins une direction dite « direction de libération » (17) pour permettre aux moyens de retenue (20) de se libérer de la partie complémentaire (22) de la prothèse (40) ;
- Des moyens de blocage (23) pouvant être placés dans deux positions :
∘ Une position fermée dans laquelle ils empêchent la déformation de la dent (16) dans la direction de libération (17);
∘ Une position ouverte dans laquelle ils n'empêchent pas la déformation de la dent (16) dans la direction de libération (17).

3. Dispositif de pose et de dépose (1) selon la revendication 2, dans lequel les moyens de retenue (20) comportent une excroissance (21) apte à se loger dans la partie complémentaire (22) de la prothèse (40).

4. Dispositif de pose et de dépose (1) selon la revendication 3, dans lequel les moyens de blocage (23) sont formés par une bague (24).

5. Dispositif de pose et de dépose (1) selon l'une des revendications 2 à 4, le dispositif de pose et de dépose (1) s'étendant suivant un axe de référence (6), les moyens de blocage (23) pouvant se déplacer longitudinalement pour passer de la position fermée à la position ouverte, les moyens de contrôle (4) comportant un curseur (10) qui peut se déplacer longitudinalement, les moyens de liaison (5) comportant un tube de liaison (29) qui relie le curseur (10) aux moyens de blocage (23) de façon à déplacer longitudinalement les moyens de blocage (23) lorsque le curseur (10) se déplace longitudinalement.

6. Dispositif de pose et de dépose (1) selon l'une des revendications précédentes, **caractérisé en ce qu'**il est en outre pourvu de moyens de guidage (15) dans lesquels un guide filaire (31) peut être inséré.

7. Dispositif de pose et de dépose (1) selon la revendication 6, dans lequel le tube de liaison (29) entoure les moyens de guidage (15).

8. Dispositif de pose et de dépose (1) selon la revendication 7, dans lequel les moyens de liaison (5) et les moyens de guidage (15) forment un tube flexible entre les moyens de préhension (3) et les moyens de fixation (2).

9. Dispositif de pose et de dépose (1) selon l'une des revendications 1 à 8, dans lequel les moyens de fixation (2) comportent en outre des moyens d'orientation (25) aptes à orienter en rotation la prothèse (40).

10. Kit pour la pose et de dépose d'une prothèse comportant :
- Un dispositif de pose et de dépose (1) selon l'une des revendications 1 à 9 ;
- Un guide filaire (31).

## Patentansprüche

1. Vorrichtung zur Passung und Entfernung (1) einer Prothese (40) auf endobukkalem Weg, umfassend:
- Befestigungsmittel (2), die geeignet sind, sich auf der Prothese (40) zu befestigen, wobei die Befestigungsmittel (2) in zwei Positionen angeordnet werden können:
* einer geschlossenen Position, in der sie mit der Prothese (40) verbunden sind;
* einer offenen Position, in der sie nicht mit der Prothese verbunden sind;
- Greifmittel (3), die die Betätigung der Vorrichtung zur Passung und Entfernung (1) ermöglichen;
- Kontrollmittel (4), wobei die Kontrollmittel (4) mit den Befestigungsmitteln (2) verbunden sind, wobei die Kontrollmittel (4) geeignet sind, die Befestigungsmittel (2) zu steuern, um sie in der geschlossenen Position oder in der offenen Position anzuordnen;
- Verbindungsmittel (5), die zumindest teilweise flexibel sind, die die Kontrollmittel (4) mit den Befestigungsmitteln (2) verbinden, um die Steuerung der Kontrollmittel (4) auf die Befestigungsmittel (2) zu übertragen, wobei sich diese Verbindungsmittel (5) bei der Einführung der Prothese (40) auf endobukkalem Weg verformen.

2. Vorrichtung zur Passung und Entfernung (1) nach Anspruch 1, bei der die Befestigungsmittel (2) umfassen:
- eine Verbindungszone (41), die geeignet ist, mit einem Teil der Prothese (40) verbunden zu werden;
- einen elastisch verformbaren Zahn (16), der mit Haltemitteln (20) versehen ist, die geeignet sind, durch einen komplementären Teil (22) der Prothese (40) gehalten zu werden, wobei sich der Zahn (16) elastisch in mindestens eine so genannte "Freigaberichtung" (17) verformen muss, um es den Haltemitteln (20) zu ermöglichen, sich aus dem komplementären Teil (22) der Prothese (40) zu lösen;
- Feststellungsmittel (23), die in zwei Positionen angeordnet werden können:
* einer geschlossenen Position, in der sie die Verformung des Zahns (16) in Freigaberichtung (17) verhindern;
* einer offenen Position, in der sie die Verformung des Zahns (16) in Freigaberichtung (17) nicht verhindern.

3. Vorrichtung zur Passung und Entfernung (1) nach Anspruch 2, bei der die Haltemittel (20) eine Ausstülpung (21) umfassen, die geeignet ist, sich in dem komplementären Teil (22) der Prothese (40) anzuordnen.

4. Vorrichtung zur Passung und Entfernung (1) nach Anspruch 3, bei der die Feststellungsmittel (23) von einem Ring (24) gebildet sind.

5. Vorrichtung zur Passung und Entfernung (1) nach einem der Ansprüche 2 bis 4, wobei sich die Vorrichtung zur Passung und Entfernung (1) entlang einer Referenzachse (6) erstreckt, wobei sich die Feststellungsmittel (23) längs verschieben können, um von der geschlossenen Position in die offene Position überzugehen, wobei die Kontrollmittel (4) einen Cursor (10) umfassen, der sich längs verschieben kann, wobei die Verbindungsmittel (5) eine Verbindungsröhre (29) umfassen, die den Cursor (10) mit den Feststellungsmitteln (23) verbindet, um die Feststellungsmittel (23) längs zu verschieben, wenn sich der Cursor (10) längs verschiebt.

6. Vorrichtung zur Passung und Entfernung (1) nach einem der vorhergehenden Ansprüche, **dadurch gekennzeichnet, dass** sie ferner mit Führungsmitteln (15) versehen ist, in die eine Drahtführung (31) eingesetzt werden kann.

7. Vorrichtung zur Passung und Entfernung (1) nach Anspruch 6, bei der die Verbindungsröhre (29) die Führungsmittel (15) umgibt.

8. Vorrichtung zur Passung und Entfernung (1) nach Anspruch 7, bei der die Verbindungsmittel (5) und die Führungsmittel (15) eine flexible Röhre zwischen den Greifmitteln (3) und den Befestigungsmitteln (2) bilden.

9. Vorrichtung zur Passung und Entfernung (1) nach einem der Ansprüche 1 bis 8, bei der die Befestigungsmittel (2) ferner Ausrichtungsmittel (25) umfassen, die geeignet sind, die Prothese (40) in Drehung auszurichten.

10. Einheit zur Passung und Entfernung einer Prothese, umfassend:
- eine Vorrichtung zur Passung und Entfernung (1) nach einem der Ansprüche 1 bis 9;
- eine Drahtführung (31).

## Claims

1. Device (1) for fitting and removing a prosthesis (40) by the oral route, said device (1) comprising:
- attachment means (2) that can be attached to the prosthesis (40), the attachment means (2) being able to be arranged in two positions:
• a closed position, in which they are rigidly connected to the prosthesis (40);
• an open position, in which they are not rigidly connected to the prosthesis;
- gripping means (3) for manipulation of the fitting and removing device (1);
- control means (4), the control means (4) being connected to the attachment means (2), the control means (4) being able to control the attachment means (2) in such a way as to place same in the closed position or the open position;
- at least partially flexible connecting means (5) that connect the control means (4) to the attachment means (2) in such a way as to transmit the command from the control means (4) to the attachment means (2), these connecting means (5) deforming during the introduction of the prosthetsis (40) by the oral route.

2. Fitting and removing device (1) according to Claim 1, in which the attachment means (2) have:
- an assembly zone (41) that can be joined to a part of the prosthesis (40);
- an elastically deformable tooth (16) provided with retention means (20) that are able to be retained by a complementary part (22) of the prosthesis (40), the tooth (16) having to deform elastically in at least one direction called the "release direction" (17) in order to allow the retention means (20) to free themselves from the complementary part (22) of the prosthesis (40);
- blocking means (23) that can be placed in two positions:
• a closed position, in which they prevent the deformation of the tooth (16) in the release direction (17);
• an open position, in which they do not prevent the deformation of the tooth (16) in the release direction (17).

3. Fitting and removing device (1) according to Claim 2, in which the retention means (20) have a protrusion (21) that can be received in the complementary part (22) of the prosthesis (40).

4. Fitting and removing device (1) according to Claim 3, in which the blocking means (23) are formed by a ring (24).

5. Fitting and removing device (1) according to one of Claims 2 to 4, the fitting and removing device (1) extending along a reference axis (6), the blocking means (23) being able to move longitudinally so as to pass from the closed position to the open position, the control means (4) having a cursor (10) that can move longitudinally, the connecting means (5) having a connecting tube (29) which connects the cursor (10) to the blocking means (23) in such a way as to move the blocking means (23) longitudinally when the cursor (10) moves longitudinally.

6. Fitting and removing device (1) according to one of the preceding claims, **characterized in that** it is additionally provided with guide means (15) in which a guide wire (31) can be inserted.

7. Fitting and removing device (1) according to Claim 6, in which the connecting tube (29) surrounds the guide means (15).

8. Fitting and removing device (1) according to Claim 7, in which the connecting means (5) and the guide means (15) form a flexible tube between the gripping means (3) and the attachment means (2).

9. Fitting and removing device (1) according to one of Claims 1 to 8, in which the attachment means (2) additionally have orientation means (25) that are able to orient the prosthesis (40) in rotation.

10. Kit for fitting and removing a prosthesis, said kit having:
- a fitting and removing device (1) according to one of Claims 1 to 9;
- a guide wire (31).
